# Europäisches Patentamt

## European Patent Office

## Office européen des brevets

(19)

(11) Veröffentlichungsnummer: **0 217 085**

**B1**

(12) # EUROPÄISCHE PATENTSCHRIFT

(45) Veröffentlichungstag der Patentschrift:
25.10.89

(21) Anmeldenummer: 86111195.3

(22) Anmeldetag: 13.08.86

(51) Int. Cl.⁴: **A 61 B 17/58**

(54) **Osteosynthetische Druckplatte.**

(30) Priorität: 30.08.85 CH 3752/85

(43) Veröffentlichungstag der Anmeldung:
08.04.87 Patentblatt 87/15

(45) Bekanntmachung des Hinweises auf die Patenterteilung:
25.10.89 Patentblatt 89/43

(84) Benannte Vertragsstaaten:
AT BE DE FR GB SE

(56) Entgegenhaltungen:
EP-A-0 181 433
DE-C-3 442 004
DE-U-8 431 616
FR-A-2 466 977
US-A-3 463 148

(73) Patentinhaber: SYNTHES AG, Grabenstrasse 15, CH-7002 Chur (CH)

(72) Erfinder: Klaue, Kaj, Dr. med., Spiegelstrasse 64, CH- 3026 Spiegel (CH)

(74) Vertreter: Lusuardi, Werther Giovanni, Dr. Lusuardi AG Kreuzbühlstrasse 8, CH- 8008 Zürich (CH)

**Beschreibung**

Die Erfindung bezieht sich auf eine osteosynthetische Druckplatte gemäss dem Oberbegriff des Patentanspruchs 1.

Eine osteosynthetische Druckplatte dieser Art ist aus dem deutschen Gebrauchsmuster DE-U1-8 431 616.0 bekannt.

Das Anlegen von osteosynthetischen Druckplatten auf die Knochenoberfläche bewirkt früher oder später eine Schwächung des Knochens. Dabei kommt es zur Nichtheilung des Knochenbruches oder zu sekundären Brüchen. Es fehlte deshalb nicht an Versuchen osteosynthetische Druckplatten mit einer verminderten Steifigkeit, beispielsweise durch Anbringen von Quernuten, zu schaffen, doch weisen solche Platten einen ungenügenden Schienungseffekt auf.

Die Steifigkeit einer osteosynthetischen Druckplatte wird durch drei Grössen bestimmt:

- dem polaren Trägheitsmoment, welches die Torsionssteifigkeit ergibt;
- dem biaxialen Trägheitsmoment (max. und min.), das die Biegesteifigkeit ergibt; und
- der zur Längsrichtung orthogonal stehenden Querschnittsfläche, welche die Zugsteifigkeit ergibt.

Um einen genügenden Schienungseffekt zu erhalten dürfen die verschiedenen Steifigkeitswerte jedoch gewisse Minimalwerte nicht unterschreiten.

Bei der zum Stand der Technik gehörenden, osteosynthetischen Druckplatte gemäss der DE-U1-8 431 616.0 handelt es sich um eine spezielle, mehrteilige osteosynthetische Druckplatte, welche gemäss der gestellten Aufgabe, zum Zwecke der Anpassung an Relativverschiebungen der Knochenfraktursegmente, neben der eigentlichen Grundplatte eine daran längsverschiebbar angebrachte Schieberplatte aufweist. Um nun für die Aufnahme der Schieberplatte den erforderlichen Platz zu schaffen ist die Grundplatte als im Querschnitt U-förmig ausgebildete Rinne ausgebildet. Die rinnenförmige Aushöhlung und U-förmige Querschnittsgestaltung der Druckplatte erfolgt somit einzig zur Aufnahme der Schieberplatte. Eine solche im Querschnitt geschwächte Knochenplatte weist jedoch wie oben dargelegt eine nur ungenügende Steifigkeit, insbesondere Torsionssteifigkeit auf, welche aus Stabilitätsgründen unbedingt zu fordern ist. Aufgabe der Erfindung ist, eine osteosynthetische Druckplatte der eingangs genannten Art zu schaffen, die unter Beibehaltung der für einen genügenden Schienungseffekt erforderlichen Steifigkeit eine verbesserte Durchblutung des Knochens ermöglicht.

Erfindungsgemäss wird diese Aufgabe durch die im kennzeichnenden Teil des Patentanspruchs 1 angeführten Merkmale gelöst. Unter der Knochenapplikationsfläche ist dabei die im wesentlichen unmittelbar an den Knochen angrenzende Fläche der Druckplatte zu verstehen.

Der Abstand zwischen Knochenapplikationsfläche und Knochen bewegt sich dabei vorzugsweise im Bereich unter 50 Mikron. Wie nachstehend durch Ausführungsbeispiele des Erfindungsgegenstandes anhand der Zeichnungen erläutert, behält die erfindungsgemässe Druckplatte die Vorteile von konventionellen Platten hoher Steifigkeit, entsprechend dem weit verbreiteten rechteckigen Profil von 12 x 3,9 mm, ohne deren Nachteile, welche sich durch die großflächige Überdachung des Knochens ergibt, aufzuweisen. Es wurde überraschenderweise gefunden, dass bei einer Verlagerung des Versteifungsmaterials der Druckplatte vom Knochen weg, die dadurch bewirkte Reduktion der Knochenüberdachung eine Verminderung der Durchblutungsstörungen und der Osteoporose zur Folge hat, was wiederum zu einer besseren Heilung und Verhinderung von Sekundärbrüchen führt. Die verminderte Kontaktbreite zwischen Druckplatte und Knochen ergibt zudem eine Übertragung der Anpresskraft vom Schraubenkopf über die Druckplatte zum Knochen nahe an der Schraubenachse. Daher treten nur minimal störende Torsionseffekte auf den Knochen auf, bedingt durch die Steifigkeit der Druckplatte

Da die Knochenapplikationsfläche nicht durch Nuten oder ähnliche Ausnehmungen unterbrochen ist bleibt der Schienungseffekt der Druckplatte auf ihrer gesamten Länge erhalten. Die Neigung von 40° der Seitenflächen der Druckplatte ergibt nach erfolgter Knochenheilung seitlich weitwinklige Knochenlamellen, wodurch nach Entfernung des Implantates eine minimale Stresskonzentration auf dem Knochen resultiert. Überdies ist auch die Entfernung der Druckplatte einfacher. Es zeigt:

Fig. 1 eine Aufsicht auf die untere, dem Knochen zugewandte Fläche der osteosynthetischen Druckplatte gemäss der Erfindung;

Fig. 2 einen zur Längsachse der osteosynthetischen Druckplatte orthogonalen Profilquerschnitt X - X von Fig. 1; und

Fig. 3 einen zur Längsachse der osteosynthetischen Druckplatte orthogonalen Profilquerschnitt Y - Y von Fig. 1.

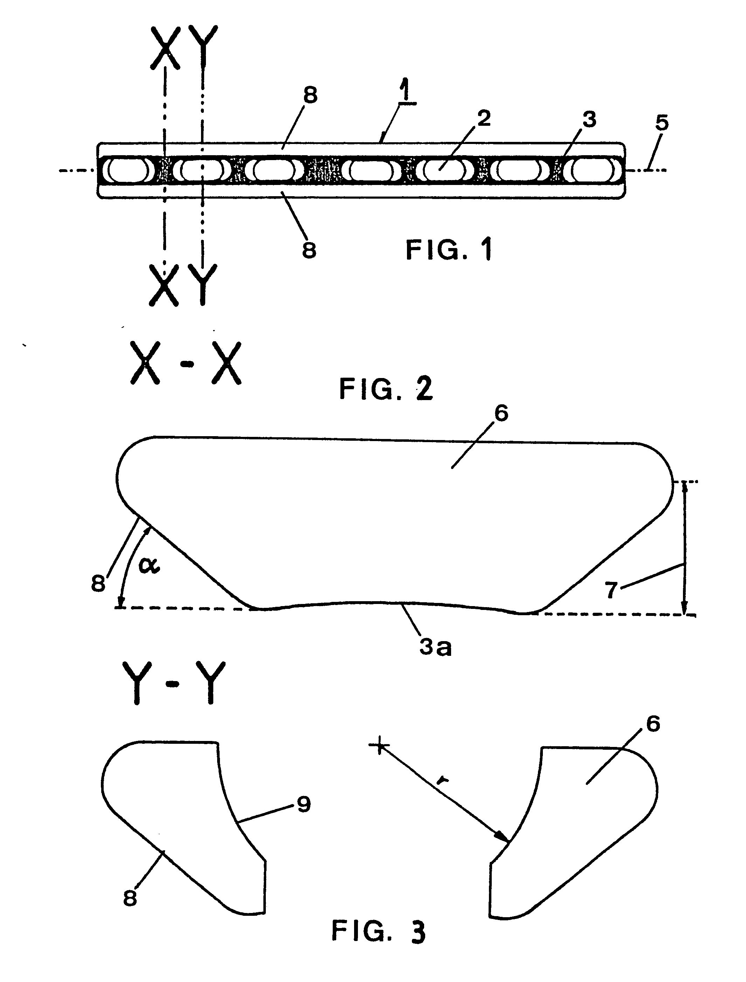

Fig. 1 zeigt die erfindungsgemässe osteosynthetische Druckplatte von unten, d.h. von der dem Knochen zugewandten Seite her. Sie weist einen Anzahl in Richtung der Längsachse 5 angeordnete Löcher 2 auf, welche zur Aufnahme von hier nicht dargestellten Knochenschrauben zur Fixierung der Druckplatte 1 am Knochen bestimmt sind. Die auf ein Minimum reduzierte Knochenapplikationsfläche 3 ist dabei als schwarze Fläche dargestellt. Die daran anschliessenden Seitenflächen 8 der Druckplatte 1 schliessen mit der Knochenapplikationsfläche 3 einen Winkel α von 40° ein, wie in Fig. 2 dargestellt. Je nach Anwendungsgebiet kann dieser Winkel α innerhalb eines Bereiches von 10 - 70°, vorzugsweise von 30 - 50° angepasst werden. Der in Fig. 2 dargestellte Profilquerschnitt zeigt deutlich die Verlagerung des Steifigkeitsmaterials vom Knochen weg. Durch diese Verlagerung, welche die Steifigkeit der Druckplatte 1 gegenüber konventionellen Druckplatten unverändert lässt, wird die Knochenapplikationslinie 3a minimalisiert und kann im Extremfall auf einen Punkt reduziert werden.

Der in Fig. 3 gezeigte Profilquerschnitt 6 auf der Höhe Y - Y der Fig. 1 ist durch das Zentrum eines Loches 2 gelegt und zeigt die übliche sphärische Auflagefläche 9 für die darin aufzunehmende Knochenschraube.

Der Profilquerschnitt 6 erweitert sich dabei kontinuierlich mit zunehmendem Abstand von der Knochenapplikationslinie 3a in Form eines Trapezes innerhalb des Teilbereiches 7. Aus konstruktiven Gründen erfolgt ausserhalb dieses Teilbereiches 7 wiederum eine Verengung des Profilquerschnitts 6.

Es sind eine ganze Reihe weiterer Modifikationen der erfindungsgemässen osteosynthetischen Druckplatte 1 möglich, beispielsweise was die Ausbildung der Knochenapplikationslinie 3a betrifft, welche wie in Fig. 2 angedeutet auch konkav ausgebildet sein kann, oder bezüglich des Profilquerschnitts 6 der sich auch diskontinuierlich erweitern kann.


## Patentansprüche

1. Osteosynthetische, einstückig ausgebildete Druckplatte (1) mit mehreren in Richtung der Plattenlängsachse (5) angeordneten Löchern (2) für die Aufnahme von Senk-Knochenschrauben, dadurch gekennzeichnet, dass die orthogonal zur Plattenlängsachse (5) stehende Querschnittsfläche (6) der Druckplatte (1) mindestens in einem Teilbereich (7) der Plattenlänge trapezoid ausgebildet ist, wobei die kürzere Grundlinie der trapezoiden Querschnittsfläche (6) der Knochenapplikationslinie (3a) entspricht, die sich als Schnittlinie zwischen der Knochenapplikationsfläche (3) und der orthogonal zur Plattenlängsachse (5) stehenden Querschnittsfläche (6) ergibt.

2. Osteosynthetische Druckplatte (1) nach Anspruch 1, dadurch gekennzeichnet, dass die an die Knochenapplikationsfläche (3) anschliessenden nichtparallelen Seitenflächen (8) der trapezoid ausgebildeten Querschnittsfläche der Druckplatte (1) mit der Kochenapplikationsfläche (3) einen Aussenwinkel von 10° - 70°, vorzugsweise von 30° - 50° einschliessen.

3. Osteosynthetische Druckplatte (1) nach Anspruch 1 oder 2, dadurch gekennzeichnet, dass die Erweiterung des Querschnitts (6) kontinuierlich erfolgt.

4. Osteosynthetische Druckplatte (1) nach Anspruch 1 oder 2, dadurch gekennzeichnet, dass die Erweiterung des Querschnitts (6) diskontinuierlich erfolgt.

5. Osteosynthetische Druckplatte (1) nach einem der Ansprüche 1 bis 4, dadurch gekennzeichnet, dass die Knochenapplikationslinie (3a) zumindest teilweise zu einem Punkt degeneriert ist.

6. Osteosynthetische Druckplatte (1) nach einem der Ansprüche 1 bis 4, dadurch gekennzeichnet, dass die Knochenapplikationsfläche (3) zu einer segmentartigen, gebrochenen Linie degeneriert ist.

7. Osteosynthetische Druckplatte (1) nach einem der Ansprüche 1 bis 4, dadurch gekennzeichnet, dass die Knochenapplikationsfläche (3) zu einer Reihe von Punkten degeneriert ist, welche sich auf einer Geraden befinden.


## Claims

1. Osteosynthetic single-piece compression plate (1) with a plurality of holes (2) disposed along the longitudinal axis (5) of the compression plate (1) for the insertion of bone screws, characterized in that the cross-sectional area (6) of the compression plate (1), which is orthogonal to the longitudinal axis (5) of the plate (1), has, at least in a segment (7) of the plate length, a trapezoidal form, whereby the shorter basis of the trapezoidal cross-sectional area (6) corresponds to the bone application line (3a), which is formed by the intersection of the bone application surface (3) and the cross-sectional area (6) orthogonal to the longitudinal axis (5) of the plate (1).

2. Osteosynthetic compression plate (1) according to claim 1, characterized in that the non-parallel, lateral surfaces (8) adjacent to the bone application surface (3) of the trapezoidal cross-section of the compression plate (1) are forming an exterior angle with the bone application surface (3) in the range from 10° - 70°, preferably from 30° - 50°.

3. Osteosynthetic compression plate (1) according to claim 1 or 2, characterized in that the enlargement of the cross-sectional area (6) is continuous.

4. Osteosynthetic compression plate (1) according to claim 1 or 2, characterized in that the enlargement of the cross-sectional area (6) is discontinuous.

5. Osteosynthetic compression plate (1) according to one of the claims 1 to 4, characterized in that the bone application line (3a) is degenerated - at least partially - to a point.

6. Osteosynthetic compression plate (1) according to one of the claims 1 to 4, characterized in that the bone application surface (3) is degenerated to a segment-like, broken line.

7. Osteosynthetic compression plate (1) according to one of the claims 1 to 4, characterized in that the bone application surface (3) is degenerated to a series of points which are disposed along a straight line.

**Revendications**

1. Plaque compressive (1) d'ostéosynthèse, réalisée en une seule pièce, comportant plusieurs trous (2) agencés en direction de l'axe longitudinal (5) pour la réception de vis à tête conique ou fraisée pour les os, caractérisé en ce que la surface transversale (6) de la plaque compressive (1) située orthogonalement par rapport à l'axe longitudinal (5) de la plaque, est réalisée de manière trapézoïdale dans au moins une zone partielle (7) de la longueur de la plaque, si bien que la ligne de base plus courte de la surface transversale trapézoïdale (6) correspond à la ligne d'application aux os (3a) et se comporte comme une ligne d'intersection entre la surface d'application aux os (3) et la surface transversale (6) situé orthogonalement par rapport à l'axe longitudinal (5) de la plaque.

2. Plaque compressive (1) d'ostéosynthèse selon la revendication 1, caractérisée en ce que les parois latérales (8), non parallèles, qui se raccordent à la surface d'application (3) aux os, de la surface transversale de conformation trapézoïdale de la plaque compressive (1) incluent un angle externe de 10 - 70°, de préférence de 30 - 50°, avec la surface d'application (3) aux os.

3. Plaque compressive (1) d'ostéosynthèse selon la revendication 1 ou 2, caractérisée en ce que l'élargissement de la section transversale (6) est continu.

4. Plaque compressive (1) d'ostéosynthèse suivant la revendication 1 ou 2, caractérisée en ce que l'élargissement de la section transversale (6) est discontinu.

5. Plaque compressive (1) d'ostéosynthèse suivant l'une quelconque des revendications 1 à 4, caractérisée en ce que la ligne d'application aux os (3a) est dégénérée au moins partiellement en un point.

6. Plaque compressive (1) d'ostéosynthèse suivant l'une quelconque des revendications 1 à 4, caractérisée en ce que la surface d'application (3) aux os est dégénérée en une ligne en traits interrompus, segmentaire.

7. Plaque compressive (1) d'ostéosynthèse selon l'une quelconque des revendications 1 à 4, caractérisée en ce que la surface d'application (3) aux os est dégénérée en une série de points qui se trouvent sur une droite.

X Y

8  1  2  3  5

X  Y

**FIG. 1**

X X

X - X

**FIG. 2**

6

8  α

7

3a

Y - Y

6

9

r

8

**FIG. 3**

1